(19) Europäisches Patentamt / European Patent Office / Office européen des brevets

(11) **EP 3 198 301 B1**

(12) **FASCICULE DE BREVET EUROPEEN**

(45) Date de publication et mention de la délivrance du brevet:
**21.04.2021 Bulletin 2021/16**

(21) Numéro de dépôt: **15781123.3**

(22) Date de dépôt: **25.09.2015**

(51) Int Cl.:
*G01S 15/89* *(2006.01)*        *G01S 7/52* *(2006.01)*
*G10K 11/34* *(2006.01)*

(86) Numéro de dépôt international:
**PCT/FR2015/052566**

(87) Numéro de publication internationale:
**WO 2016/046506 (31.03.2016 Gazette 2016/13)**

(54) **PROCÉDÉ ET DISPOSITIF D'IMAGERIE ACOUSTIQUE**

VERFAHREN UND VORRICHTUNG FÜR AKUSTISCHE BILDGEBUNG

ACOUSTIC IMAGING METHOD AND DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.09.2014 FR 1459136**

(43) Date de publication de la demande:
**02.08.2017 Bulletin 2017/31**

(73) Titulaires:
• **Centre National de la Recherche Scientifique - CNRS**
  **75016 Paris (FR)**
• **INSERM (Institut National de la Santé et de la Recherche Médicale)**
  **75013 Paris (FR)**

(72) Inventeurs:
• **TANTER, Mickaël**
  **F-92220 Bagneux (FR)**
• **OSMANSKI, Bruno-Félix**
  **F-75013 Paris (FR)**
• **PERNOT, Mathieu**
  **F-75004 Paris (FR)**
• **GENNISSON, Jean-Luc**
  **F-95000 Cergy (FR)**

(74) Mandataire: **Osha Liang**
  **2, rue de la Paix**
  **75002 Paris (FR)**

(56) Documents cités:
**WO-A1-2013/069752      US-A- 5 851 187**
**US-A- 6 048 315**

• CHIAO R Y ET AL: "Sparse array imaging with spatially-encoded transmits", ULTRASONICS SYMPOSIUM, 1997. PROCEEDINGS., 1997 IEEE TORONTO, ONT., CANADA 5-8 OCT. 1997, NEW YORK, NY, USA,IEEE, US, vol. 2, 5 octobre 1997 (1997-10-05), pages 1679-1682, XP010271620, DOI: 10.1109/ULTSYM.1997.663318 ISBN: 978-0-7803-4153-1
• SVETOSLAV I NIKOLOV AND JORGEN A JENSEN: "Comparison Between Di erent Encoding Schemes for Synthetic Aperture Imaging", PROGRESS IN BIOMEDICAL OPTICS AND IMAGING, SPIE - INTERNATIONAL SOCIETY FOR OPTICAL ENGINEERING, BELLINGHAM, WA, US, vol. 4687, 1 janvier 2002 (2002-01-01), pages 1-12, XP007906293, ISSN: 1605-7422 ISBN: 978-1-62841-502-5
• MONTALDO G ET AL: "Coherent plane-wave compounding for very high frame rate ultrasonography and transient elastography", IEEE TRANSACTIONS ON ULTRASONICS, FERROELECTRICS AND FREQUENCY CONTROL, IEEE, US, vol. 56, no. 3, 1 mars 2009 (2009-03-01), pages 489-506, XP011255897, ISSN: 0885-3010 cité dans la demande

**Description**

DOMAINE DE L'INVENTION

La présente invention est relative aux procédés et dispositifs d'imagerie acoustique.

ARRIERE-PLAN DE L'INVENTION

Le document EP2101191 et l'article de Montaldo et al. « Coherent plane-wave compounding for very high frame rate ultrasonography and transient elastography » (IEEE Trans Ultrason Ferroelectr Freq Control 2009 Mar ; 56(3): 489-506), décrivent un procédé d'imagerie acoustique, dans lequel on fait successivement émettre dans un milieu à imager, par un réseau de transducteurs acoustiques, une série de n ondes incidentes $E_i(t)$ acoustiques planes ou divergentes, les n ondes incidentes étant propagées dans le milieu à imager et générant respectivement n ondes réverbérées, et on fait capter par le réseau de transducteurs, des signaux représentatifs des ondes réverbérées.

RESUME DE L'INVENTION

Ce procédé a permis des progrès considérables en termes de rapidité d'acquisition de l'image, et de définition d'image.

Les procédés de ce type présentent toutefois l'inconvénient que la sensibilité des images obtenues diminue rapidement avec la profondeur du fait de l'atténuation des ondes ultrasonores dans le milieu à imager, notamment des tissus biologiques.

Une solution à ce problème est d'augmenter l'amplitude des ondes ultrasonores émises. Or, cette amplitude ne peut pas être augmentée au-delà de certains seuils, dépendant des transducteurs ultrasonores et / ou de l'électronique qui commande les transducteurs et / ou des normes de sécurité en vigueur pour ce qui concerne les applications médicales.

D'autres solutions basées sur un codage temporel ou spatial des signaux émis ont été proposées, mais ces solutions nécessitent l'utilisation d'électroniques d'émissions complexes et couteuses, qui ne peuvent en pratique pas être utilisés sur des appareils d'imagerie échographique classiques.

La présente invention a notamment pour but de pallier ces inconvénients et de proposer un procédé d'imagerie acoustique permettant une sensibilité améliorée sans perte de cadence d'imagerie, sans augmenter notablement la complexité ou le coût des appareils d'imagerie acoustique sur lesquels il est mis en œuvre.

A cet effet, la présente invention propose un procédé d'imagerie acoustique comprenant les étapes suivantes :

(a) une étape d'émission / réception au cours de laquelle on fait successivement émettre dans une zone d'observation appartenant à un milieu à imager, par un réseau de transducteurs acoustiques, une série de n ondes acoustiques incidentes $E_i(t)$ et on fait capter par le réseau de transducteurs acoustiques, n ondes réverbérées $R_i(t)$ qui sont réverbérées respectivement par le milieu à imager suite à l'émission des n ondes incidentes, n étant un entier naturel au moins égal à 2, lesdites ondes acoustiques incidentes étant obtenues en combinant linéairement n ondes incidentes élémentaires $E0_i(t)$ de façon correspondante à la formule :

$$\vec{E}(t) = H_c . \vec{E}0(t) \qquad (1)$$

où :

$H_c$ est une matrice carrée d'ordre n (dite matrice de codage),
$\vec{E}(t) = [E_i(t)]$ est un vecteur ayant pour composantes les n ondes incidentes,
$\vec{E}0(t) = [E0_i(t)]$ est un vecteur ayant pour composantes les n ondes incidentes élémentaires, lesdites ondes incidentes élémentaires étant soit des ondes planes, soit des ondes divergentes, soit des ondes focalisées en différents points de l'espace présentant respectivement n fronts d'ondes différents,

chaque onde incidente élémentaire étant émise par une pluralité de transducteurs acoustiques du réseau de transducteurs acoustiques et balayant toute la zone d'observation,
(b) une étape de décodage au cours de laquelle on détermine n ondes réverbérées élémentaires $R0_i(t)$ en combinant linéairement les n ondes réverbérées $R_i(t)$ captées, de façon correspondante à la formule :

$$\vec{R}0(t) = \lambda . H_d . \vec{R}(t) \qquad (2)$$

où :

λ est une constante non nulle,

$H_d$ est une matrice carrée d'ordre n (dite matrice de décodage), les matrices $H_c$ et $H_d$ étant telles que Hc.Hd=D, où D est une matrice diagonale d'ordre n dont tous les éléments diagonaux $d_{ii}$ sont non nuls,

$\overline{R}(t) = [R_i(t)]$ est un vecteur ayant pour composantes les n ondes réverbérées,

$\overline{R}0(t) = [R0_i(t)]$ est un vecteur ayant pour composantes les n ondes réverbérées élémentaires,

(c) une étape de construction d'image, au cours de laquelle on réalise une image synthétique à partir des n ondes réverbérées élémentaires $R0_i(t)$.

L'invention permet ainsi d'augmenter notablement la sensibilité des images échographiques obtenues, sans avoir besoin d'augmenter le nombre de tirs d'ondes incidentes (et donc sans baisser la cadence d'images), sans avoir besoin d'augmenter la complexité de l'appareil d'échographie, et sans avoir besoin d'augmenter l'amplitude des ondes acoustiques incidentes.

En effet, les n ondes réverbérées élémentaires $R0_i(t)$ sont représentatives respectivement des signaux qui seraient obtenus en émettant séparément les n ondes incidentes élémentaires $E0_i(t)$ puis en captant les ondes réverbérées correspondantes, mais lesdites n ondes réverbérées élémentaires $R0_i(t)$ ont une amplitude supérieure à l'amplitude des signaux qui seraient captés en émettant séparément les n ondes incidentes élémentaires $E0_i(t)$, ce qui induit une amélioration de la sensibilité.

Ce gain en amplitude et donc en sensibilité, est dû au fait que chaque onde réverbérée élémentaire $R0_i(t)$ combine des informations venant de n tirs d'ondes incidentes, donc est plus riche en information.

L'invention est utilisable dans tous les procédés où l'image échographique est synthétisée à partir de n tirs d'ondes planes ou divergentes ou multifocalisées, notamment tel que décrit dans le document EP2101191, par exemple pour l'imagerie des ondes de cisaillement, l'imagerie des réseaux sanguins (imagerie Doppler ultrasensible ou autre, notamment pour l'imagerie fonctionnelle du cerveau), etc.

Dans divers modes de réalisation du procédé selon l'invention, on peut éventuellement avoir recours en outre à l'une et/ou à l'autre des dispositions suivantes :

- les matrices $H_c$ et $H_d$ sont telles que Hc.Hd=D, où D est une matrice diagonale d'ordre n dont tous les éléments diagonaux $d_{ii}$ sont supérieurs à 1 ;
- n est un entier naturel pair non nul ;
- la matrice Hc est une matrice de Hadamard d'ordre n et la matrice $H_d$ est la transposée de la matrice Hc ;
- chacune desdites ondes acoustiques est représentée par une pluralité de signaux temporels, chaque signal temporel représentant ladite onde acoustique au niveau d'un transducteur appartenant audit réseau de transducteurs ;
- le procédé comporte en outre, après l'étape de décodage, une étape de construction d'image, au cours de laquelle on réalise une image synthétique à partir des n ondes réverbérées élémentaires $R0_i(t)$.

Par ailleurs, l'invention a également pour objet un dispositif pour la mise en œuvre d'un procédé tel que défini ci-dessus, comprenant:

(a) des moyens d'émission adaptés pour faire successivement émettre dans une zone d'observation appartenant à un milieu à imager, par un réseau de transducteurs acoustiques, une série de n ondes acoustiques incidentes $E_i(t)$, lesdites ondes acoustiques incidentes étant obtenues en combinant linéairement n ondes incidentes élémentaires $E0_i(t)$ de façon correspondante à la formule :

$$\vec{E}(t) = H_c . \vec{E}0(t) \qquad (1)$$

où :

$H_c$ est une matrice carrée d'ordre n,

$\overline{E}(t) = [E_i(t)]$ est un vecteur ayant pour composantes les n ondes incidentes,

$\overline{E}0(t) = [E0_i(t)]$ est un vecteur ayant pour composantes les n ondes incidentes élémentaires, lesdites ondes incidentes élémentaires étant soit des ondes planes, soit des ondes divergentes, soit des ondes focalisées en différents points de l'espace présentant respectivement n fronts d'ondes différents,

chaque onde incidente élémentaire étant émise par une pluralité de transducteurs acoustiques du réseau de transducteurs acoustiques et balayant toute la zone d'observation,
(b) des moyens de réception adaptés pour capter par le réseau de transducteurs acoustiques, successivement n ondes réverbérées $R_i(t)$ qui sont réverbérées respectivement par le milieu à imager suite à l'émission des n ondes incidentes,
(c) des moyens de décodage adaptés pour déterminer n ondes réverbérées élémentaires $R0_i(t)$ en combinant linéairement les n ondes réverbérées $R_i(t)$ captées, de façon correspondante à la formule :

$$\vec{R0}(t) = H_d . \vec{R}(t) \qquad (2)$$

où :

$H_d$ est une matrice carrée d'ordre n, les matrices $H_c$ et $H_d$ étant telles que $Hc.Hd=D$ où D est une matrice diagonale d'ordre n dont tous les éléments diagonaux $d_{ii}$ sont non nuls,
$\overline{R}(t) = [R_i(t)]$ est un vecteur ayant pour composantes les n ondes réverbérées,
$\overline{R0}(t) = [R0_i(t)]$ est un vecteur ayant pour composantes les n ondes réverbérées élémentaires,

(d) des moyens de construction d'image, adaptés pour réaliser une image synthétique à partir des n ondes réverbérées élémentaires $R0_i(t)$.

## BREVE DESCRIPTION DES DESSINS

D'autres caractéristiques et avantages de l'invention apparaîtront au cours de la description suivante d'un de ses modes de réalisation, donné à titre d'exemple non limitatif, en regard des dessins joints.
Sur les dessins :

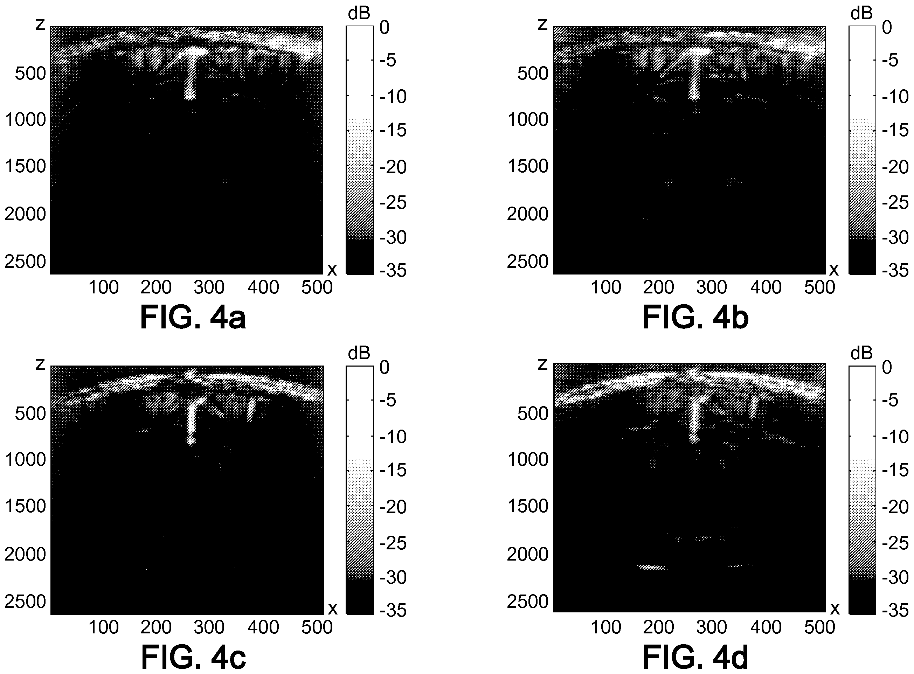

- la figure 1 est une vue schématique d'un dispositif pour la mise en œuvre d'un procédé selon un mode de réalisation de l'invention,
- la figure 2 est un schéma bloc d'une partie du dispositif de la figure 1,
- la figure 3 est une série de chronogrammes représentant les ondes incidentes élémentaires composant les différentes ondes acoustiques incidentes émises successivement pour réaliser une image synthétique,
- les figues 4a à 4d sont des images Doppler ultrarapides montrant la vascularisation d'un cerveau, les figures 4a et 4c étant réalisées dans deux plans différents par imagerie synthétique sans la présente invention, et les figures 4b et 4d étant réalisées respectivement dans les mêmes plans par imagerie synthétique avec la présente invention, et

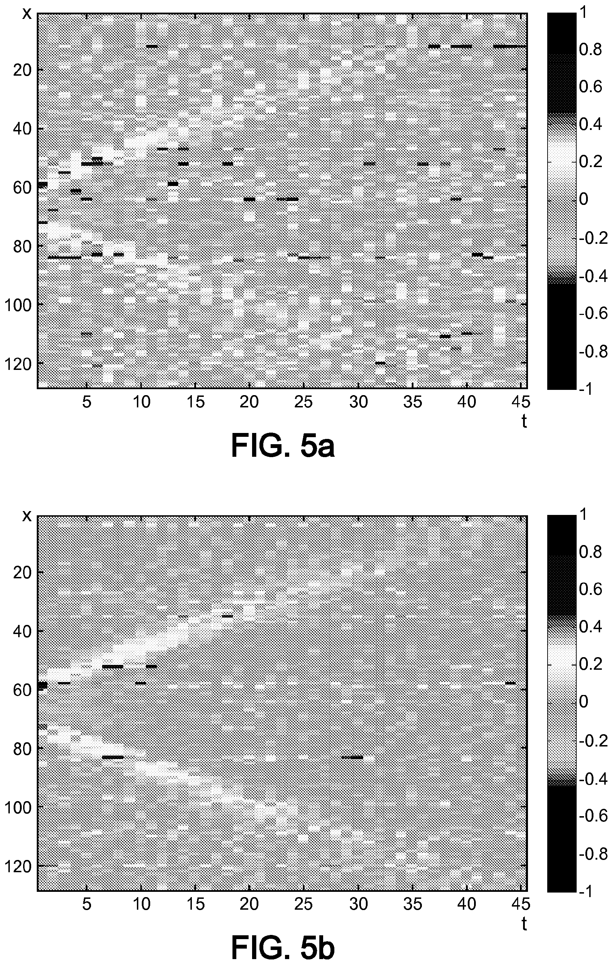

- les figures 5a et 5b sont des images élastographiques montrant la propagation d'une onde de cisaillement dans des tissus humains, obtenues par imagerie synthétique respectivement avec et sans l'invention.

## DESCRIPTION DETAILLEE

Sur les différentes figures, les mêmes références désignent des éléments identiques ou similaires.

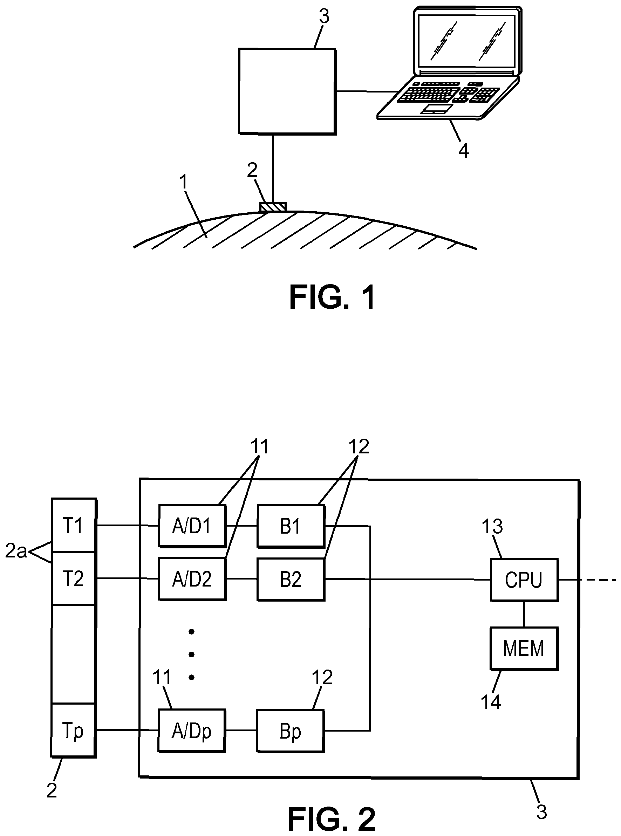

Les figures 1 et 2 montrent un exemple de dispositif adapté pour réaliser une imagerie échographique synthétique d'un milieu 1 à imager, par exemple des tissus d'un sujet humain ou animal à étudier, par émission et réception d'ondes ultrasonores de compression, par exemple de fréquences comprises entre 2 et 40 MHz, notamment entre 2 et 10 MHz. L'image échographique réalisée correspond à une portion du milieu 1 qui sera appelée ici zone d'observation.
L'émission et réception d'ondes ultrasonores peut se faire au moyen d'un réseau 2 de p transducteurs ultrasonores 2a $(T_1\text{-}T_n)$, comprenant par exemple quelques centaines de transducteurs 2a (par exemple au moins 100), ce réseau 2 pouvant être par exemple une barrette de transducteurs (réseau 1D) adapté pour réaliser une image bidimensionnelle (2D) ou encore un réseau à deux dimensions adapté pour réaliser une image tridimensionnelle (3D).
Le dispositif d'imagerie comporte également, par exemple :

- un circuit électronique 3 commandant le réseau de transducteurs 2 et adapté pour faire émettre des ondes ultrasonores par le réseau de transducteurs et acquérir les signaux captés par ce réseau de transducteurs ;
- un ordinateur 4 ou similaire pour commander le circuit électronique 3 et visualiser les images ultrasonores obtenues à partir desdits signaux captés.

Comme représenté sur la figure 2, le circuit électronique 3 peut comprendre par exemple :

- p convertisseurs analogique / digital 11 (A/D$_j$) connectés individuellement aux p transducteurs T$_1$-T$_p$ du réseau 2 de transducteurs,
- p mémoires tampon 12 (B$_j$) respectivement connectées aux p convertisseurs analogique / digital 11,
- une unité centrale 13 (CPU) communiquant avec les mémoires tampon 12 et l'ordinateur 4 et adapté au traitement des signaux envoyés vers le réseau de transducteurs 2 et reçus dudit réseau de transducteurs,
- une mémoire 14 (MEM) connectée à l'unité centrale 13.

Le dispositif d'imagerie représenté sur les figures 1 et 2 est adapté pour réaliser une imagerie ultrasonore synthétique du milieu 1 comme décrit notamment dans le document EP2101191.
On peut distinguer plusieurs étapes principales dans cette méthode d'imagerie :

(a) une étape d'émission / réception
(b) une étape de décodage,
(c) une étape de construction d'image.

Ces différentes étapes vont maintenant être détaillées. Les étapes d'émission et de décodage font appel à des matrices de codage H$_c$ et décodage H$_d$, qui seront décrites préalablement.
Ces matrices de codage H$_c$ et décodage H$_d$ sont matrices carrées d'ordre n, où n est un entier naturel au moins égale à 2, préférentiellement pair. n peut par exemple être compris entre 2 et 100, avantageusement entre 4 et 20.
Les matrices de codage H$_c$ et de décodage H$_d$ sont telles que Hc.Hd=D où D est une matrice diagonale d'ordre n dont tous les éléments diagonaux d$_{ii}$ sont non nuls.
Avantageusement, D peut être une matrice diagonale d'ordre n dont tous les éléments diagonaux d$_{ii}$ sont positifs, avantageusement supérieurs à 1.
Plus avantageusement encore, la matrice Hc peut être proportionnelle à une matrice de Hadamard H$_n$ d'ordre n et la matrice H$_d$ est la transposée de la matrice Hc.
Autrement dit :

$$H_c = \lambda.H_n \qquad (0)$$

et

$$H_d = {}^tH_c \qquad (0'),$$

Où $\lambda$ est une constante non nulle.
Des exemples de matrices de Hadamard H$_n$ sont donnés ci-après, pour n=2 et n=4.

Pour n=2 :

$$H_2 = \begin{bmatrix} 1 & 1 \\ 1 & -1 \end{bmatrix}$$

Pour n=4 :

$$H_4 = \begin{bmatrix} 1 & 1 & 1 & 1 \\ 1 & -1 & 1 & -1 \\ 1 & 1 & -1 & -1 \\ 1 & -1 & -1 & 1 \end{bmatrix}.$$

Dans le cas où la matrice de codage est une matrice de Hadamard ($H_c = H_n$), la matrice diagonale D susmentionnée est égale à n fois la matrice identité (autrement dit tous les éléments diagonaux $d_{ii}$ de D sont égaux à n).

**(a) étape d'émission / réception**

**(a1) Emissions :**

Au cours de l'étape d'émission / réception, l'unité centrale 13 fait successivement émettre dans un milieu 1, par le réseau 2 de transducteurs, une série de n ondes acoustiques incidentes $E_i(t)$, notamment des ondes ultrasonores comme décrit précédemment. Les ondes acoustiques incidentes sont généralement des impulsions de moins d'une microseconde, typiquement environ 1 à 10 cycles de l'onde ultrasonore à la fréquence centrale. Les tirs d'ondes incidentes peuvent être séparés les uns des autres par exemple d'environ 50 à 200 microsecondes.

Chacune des ondes acoustiques incidentes est obtenue en combinant linéairement n ondes incidentes élémentaires $E0_i(t)$ qui sont soit des ondes planes, soit des ondes divergentes, soit des ondes focalisées en différents points de l'espace présentant respectivement n fronts d'ondes différents.

Comme décrit dans le document EP2101191, ces ondes élémentaires sont telles qu'elles seraient émises chacune par plusieurs transducteurs (de préférence au moins 10 transducteurs, et généralement les p transducteurs du réseau 2) pour balayer toute la zone à imager dans le milieu 1.

Cette combinaison linéaire se fait de façon correspondante à la formule :

$$\vec{E}(t) = H_c . \vec{E}0(t) \qquad (1)$$

où :

    $\overline{E}(t) = [E_i(t)]$ est un vecteur ayant pour composantes les n ondes incidentes,

    $\overline{E}0(t) = [E0_i(t)]$ est un vecteur ayant pour composantes les n ondes incidentes élémentaires.

Les ondes acoustiques incidentes peuvent être calculées à l'avance et mémorisées par exemple dans la mémoire 14.

Chaque onde incidente élémentaire est émise par une pluralité de transducteurs acoustiques du réseau 2 de transducteurs acoustiques et balaye toute la zone d'observation.

Avantageusement, chacune des n ondes acoustiques incidentes $E_i(t)$ peut être représentée par une pluralité de signaux temporels $E_{ij}(t)$ représentant ladite onde acoustique incidente telle qu'émise respectivement par les différents transducteurs $T_j$ dudit réseau 2 de transducteurs. Ainsi, $E_i(t)$ peut être représentée par un vecteur $E_i(t) = [E_{ij}(t)]$.

De même, chacune des n ondes incidentes élémentaires $E0_i(t)$ peut être représentée par une pluralité de signaux temporels $E0_{ij}(t)$ représentant ladite onde incidente élémentaire respectivement au niveau des différents transducteurs $T_j$ dudit réseau 2 de transducteurs. Ainsi, $E0_i(t)$ peut être représentée par un vecteur $E0_i(t) = [E0_{ij}(t)]$.

On notera que pour une même onde émise $E_i(t)$, donc à i constant, les signaux $E_{ij}(t)$ sont tous différents les uns des autres, par leur amplitude et leur forme temporelle, le codage effectué ici étant un codage spatio-temporel.

Chaque signal $E_{ij}(t)$ émis par le transducteur j est une combinaison linéaire des signaux élémentaires $E_{ij0}(t)$,

$$\left( E_{ij}(t) = \sum_{k=1}^{n} H_{cik} . E_{kj}0(t) \right).$$

cette combinaison linéaire étant issue de la formule (1) ci-dessus La matrice Hc étant à coefficients non nuls, la combinaison linéaire susmentionnée est également à coefficients non nuls ; autrement dit, le signal $E_{ij}(t)$ émis par chaque transducteur j est une combinaison linéaire à coefficients non nuls (par exemple, +1 ou -1 dans le cas de la matrice de Hadamard) de tous les signaux élémentaires $E_{ij}0(t)$ correspondant aux différentes ondes élémentaires i (au total n ondes élémentaires).

Les signaux $E_{ij}0(t)$ sont également différents les uns des autres d'un transducteur j à l'autre par leur forme temporelle, ce qui est indispensable pour obtenir les formes d'ondes élémentaires souhaitées.

De plus, les différentes composantes $E_{ij}0(t)$ sont des signaux non nuls, de sorte que la combinaison linéaire est opérante et que chaque signal $E_{ij}(t)$ est différent du signal $E_{ij}0(t)$.

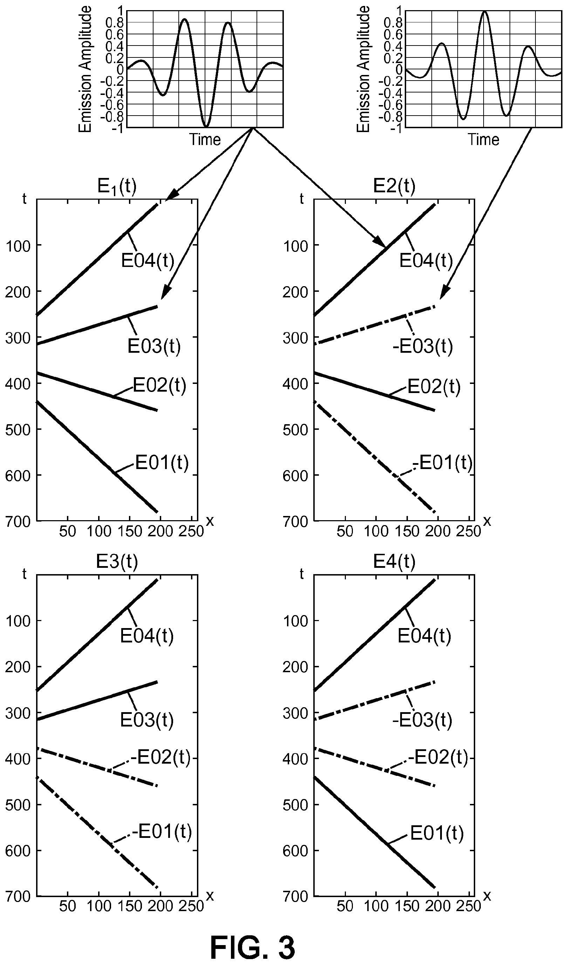

La figure 3 illustre un cas particulier avec n = 4, où les ondes acoustiques incidentes $E_i(t)$ sont des combinaisons linéaires de quatre ondes incidentes élémentaires qui sont des ondes planes $E0_i(t)$ d'inclinaisons différentes, dont les fronts d'ondes sont représentés selon l'axe X parallèle au réseau de transducteurs et en fonction du temps t. Dans cet exemple, la matrice de codage utilisée est la matrice de Hadamard $H_4$ susmentionnée.

Les ondes planes affectées d'un coefficient linéaire -1 ($-E0_i(t)$) sont représentées en traits mixtes et les ondes planes affectées d'un coefficient linéaire +1 ($+E0_i(t)$) sont représentées en traits pleins. Comme représenté sur les vues de détail en haut de la figure 3 pour les ondes $E_i(t)$ et $E_2(t)$, les formes d'ondes des ondes incidentes élémentaires $E0_i(t)$ peuvent être des sinusoïdes amorties durant par exemple environ 3 cycles, les ondes planes affectées d'un coefficient linéaire -1 ($-E0_i(t)$) ayant simplement une forme d'onde opposée à $E0_i(t)$ .

**(a2)** Réceptions :

Chacune des ondes acoustiques incidentes $E_i(t)$ se propage dans le milieu 1 qui renvoie une onde réverbérée correspondante $R_i(t)$. Avant l'émission de l'onde acoustique incidente suivante, le réseau 2 de transducteurs capte l'onde réverbérée $R_i(t)$ qui est transmise à l'unité centrale 13 et mémorisée dans la mémoire 14.

L'onde réverbérée $R_i(t)$ est mémorisée sous la forme d'une pluralité de signaux temporels $R_{ij}(t)$ représentant ladite onde réverbérée telle que captée respectivement par les différents transducteurs $T_j$ du réseau 2 de transducteurs. Ainsi, $R_i(t)$ peut être représentée par un vecteur $R_i(t) = [R_{ij}(t)]$ .

**(b) étape de décodage**

Après émission des n ondes acoustiques incidentes $E_i(t)$ et réception des n ondes réverbérées $R_i(t)$, l'unité centrale 13 procède à une étape de décodage au cours de laquelle on détermine n ondes réverbérées élémentaires $R0_i(t)$ en combinant linéairement les n ondes réverbérées $R_i(t)$ captées, de façon correspondante à la formule :

$$\vec{R0}(t) = H_d . \vec{R}(t) \qquad (2)$$

où :

$\vec{R}(t) = [R_i(t)]$ est un vecteur ayant pour composantes les n ondes réverbérées,
$\vec{R0}(t) = [R0_i(t)]$ est un vecteur ayant pour composantes les n ondes réverbérées élémentaires.

Les ondes réverbérées élémentaires $R0_i(t)$ sont déterminées sous la forme d'une pluralité de signaux temporels $R0_{ij}(t)$ représentant ladite onde réverbérée élémentaire au niveau des différents transducteurs $T_j$ du réseau 2 de transducteurs. Ainsi, $R0_i(t)$ peut être représentée par un vecteur $R0_i(t)=[R0_{ij}(t)]$, ou plus couramment par une matrice exactement $R0_i(t) = [R0_{ijk}(t_k)]$ après échantillonnage temporel en K instants $t_k$.

**(c) étape de construction d'image**

Après l'étape de décodage (b), l'unité centrale 13 procède à une étape de construction d'image, au cours de laquelle on réalise une image synthétique à partir des n ondes réverbérées élémentaires $R0_i(t)$, par exemple comme enseigné dans le document EP2101191 ou dans l'article de Montaldo et al. « Coherent plane-wave compounding for very high frame rate ultrasonography and transient elastography » (IEEE Trans Ultrason Ferroelectr Freq Control 2009 Mar ; 56(3): 489-506).

Les ondes réverbérées élémentaires $R0_i(t)$, généralement présentées sous forme matricielle $R0_i(t) = [R0_{ijk}(t_k)]$ comme indiqué précédemment, constituent les matrices de données brutes de départ (ou « données brutes RF ») des procédés décrits dans les documents susmentionnés. Plus particulièrement, à partir des n matrices $R0_i(t)$ de données brutes RF, l'unité centrale 13 calcule des matrices de données cohérentes synthétiques RF, respectivement en M points $P_m(x, z)$ du milieu à imager 1 (dits points de focalisation virtuels), m étant un entier compris entre 1 et M, x l'abscisse du point $P_m$ sur un axe X parallèle au réseau 2 de transducteurs dans le cas d'un réseau 2 linéaire de transducteurs (x étant remplacé par x,y dans le cas d'un réseau 2 bidimensionnel) et z est la profondeur dans le milieu 1, selon un axe Z perpendiculaire à l'axe X. Chacune de ces M matrices de données cohérentes synthétiques RF comporte p signaux temporels RFcoherent$_{m,j}$ (t) correspondant aux signaux qui seraient captés respectivement par les p transducteurs T$_j$ si les transducteurs émettaient une onde incidente focalisée au point P$_m$.

Les matrices de données RF cohérentes peuvent être obtenues par exemple en supposant une vitesse de propagation c homogène dans tout le milieu 1 pour les ondes de compression ultrasonores, selon le principe expliqué notamment dans le document EP2101191 ou dans l'article de Montaldo et al. « Coherent plane-wave compounding for very high frame rate ultrasonography and transient elastography » (IEEE Trans Ultrason Ferroelectr Freq Control 2009 Mar ; 56(3): 489-506).

La direction de propagation de l'onde plane correspondant à chaque one incidente élémentaire E0$_i$(t) étant connue, et la vitesse de propagation c étant connue, l'unité centrale 13 peut calculer pour chaque point P$_m$ le temps de propagation $\tau_{ec}$ (i,m) de l'onde incidente élémentaire i jusqu'au point P$_m$, et le temps de propagation $\tau_{rec}$(i,m,j) de l'onde réverbérée élémentaire depuis le point P$_m$ vers le transducteur T$_j$, donc le temps de trajet total aller-retour $\tau$ (i,m,j) = $\tau_{ec}$(i, P$_m$) + $\tau_{rec}$(i,P$_m$,j).

Le signal spatialement cohérent pour le transducteur Tj, correspondant au point de focalisation virtuel P$_m$, est alors calculé selon la formule :

$$RFcoherent_{mj}(t) = \sum_i B(i)RFraw_{ij}(\tau(i, m, j)) \qquad (3)$$

où B(i) est une fonction de pondération pour la contribution de chaque onde incidente élémentaire i (éventuellement, les coefficients B(i) peuvent être tous égaux à 1)

Les matrices de données cohérentes RFcoherent$_{mj}$ peuvent ensuite être éventuellement affinées en corrigeant les effets d'aberrations dans le milieu 1, par exemple comme expliqué dans les documents susmentionnés EP2101191 ou Montaldo et al.

L'unité centrale 13 peut alors procéder à une formation de voie en réception classique pour obtenir une image ultrasonore comme expliqué dans les documents susmentionnés EP2101191 ou Montaldo et al., et / ou procéder à des traitements plus élaborés (par exemple calcul d'une image Doppler ou Doppler couleur), connus en soi.

Le procédé de construction d'image est similaire lorsque le réseau 2 de transducteurs est bidimensionnel, ou lorsque les ondes incidentes élémentaires et les ondes réverbérées élémentaires sont divergentes.

Dans tous les cas, l'image obtenue présente une sensibilité nettement accrue par rapport à une image obtenue par imagerie synthétique classique par émission d'ondes planes (telle que décrite par les documents susmentionnés EP2101191 ou Montaldo et al).

Ainsi, les figues 4a et 4c des images Doppler ultrarapides montrant la vascularisation d'un cerveau de rat, réalisées dans deux plans différents par imagerie synthétique avec émission d'ondes planes (sans la présente invention), tandis que les figures 4b et 4d sont réalisées respectivement dans les mêmes plans par imagerie synthétique avec la présente invention : la sensibilité nettement accrue des images des figues 4b et 4d est visible à l'œil.

De même, les figures 5a et 5b sont des images élastographiques montrant la propagation d'une onde de cisaillement dans des tissus humains, obtenues par imagerie synthétique respectivement avec et sans l'invention : la sensibilité supérieure de l'image de la figure 5b est visible à l'oeil.

**Revendications**

1. Procédé d'imagerie acoustique comprenant les étapes suivantes :

(a) une étape d'émission / réception au cours de laquelle on fait successivement émettre dans une zone d'observation appartenant à un milieu (1) à imager, par un réseau (2) de transducteurs acoustiques, une série de n ondes acoustiques incidentes E$_i$(t) et on fait capter par le réseau de transducteurs acoustiques, n ondes réverbérées R$_i$(t) qui sont réverbérées respectivement par le milieu à imager suite à l'émission des n ondes incidentes, n étant un entier naturel au moins égal à 2, lesdites ondes acoustiques incidentes étant obtenues en combinant linéairement n ondes incidentes élémentaires E0$_i$(t) de façon correspondante à la formule :

$$\vec{E}(t) = H_c . \vec{E}0(t) \qquad (1)$$

où :

H$_c$ est une matrice carrée d'ordre n,

$\vec{E}(t) = [E_i(t)]$ est un vecteur ayant pour composantes les n ondes incidentes,

$\vec{E}0(t) = [E0_i(t)]$ est un vecteur ayant pour composantes les n ondes incidentes élémentaires, lesdites ondes incidentes élémentaires étant soit des ondes planes, soit des ondes divergentes, soit des ondes focalisées en différents points de l'espace, présentant respectivement n fronts d'ondes différents,

chaque onde incidente élémentaire étant émise par une pluralité de transducteurs acoustiques du réseau (2) de transducteurs acoustiques et balayant toute la zone d'observation,

(b) une étape de décodage au cours de laquelle on détermine n ondes réverbérées élémentaires $R0_i(t)$ en combinant linéairement les n ondes réverbérées $R_i(t)$ captées, de façon correspondante à la formule :

$$\vec{R}0(t) = H_d . \vec{R}(t) \qquad (2)$$

où :

$H_d$ est une matrice carrée d'ordre n, les matrices $H_c$ et $H_d$ étant telles que Hc.Hd=D où D est une matrice diagonale d'ordre n dont tous les éléments diagonaux $d_{ii}$ sont non nuls,

$\vec{R}(t) = [R_i(t)]$ est un vecteur ayant pour composantes les n ondes réverbérées,

$\vec{R}0(t) = [R0_i(t)]$ est un vecteur ayant pour composantes les n ondes réverbérées élémentaires,

(c) une étape de construction d'image, au cours de laquelle on réalise une image synthétique à partir des n ondes réverbérées élémentaires $R0_i(t)$.

2. Procédé selon la revendication 1, dans lequel les matrices $H_c$ et $H_d$ sont telles que Hc.Hd=D, où D est une matrice diagonale d'ordre n dont tous les éléments diagonaux $d_{ii}$ sont supérieurs à 1.

3. Procédé selon la revendication 1 ou la revendication 2, dans lequel n est un entier naturel pair non nul.

4. Procédé selon l'une quelconque des revendications précédentes, dans lequel la matrice Hc est une matrice de Hadamard d'ordre n et la matrice $H_d$ est la transposée de la matrice Hc.

5. Procédé selon l'une quelconque des revendications précédentes, dans lequel chacune desdites ondes acoustiques est représentée par une pluralité de signaux temporels, chaque signal temporel représentant ladite onde acoustique au niveau d'un transducteur appartenant audit réseau de transducteurs.

6. Procédé selon l'une quelconque des revendications précédentes, dans lequel le milieu (1) à imager comprend un tissu biologique vivant humain ou animal.

7. Dispositif d'imagerie acoustique, comprenant:

(a) des moyens d'émission (13, 2) adaptés pour faire successivement émettre dans une zone d'observation appartenant à un milieu (1) à imager, par un réseau (2) de transducteurs acoustiques, une série de n ondes acoustiques incidentes $E_i(t)$, lesdites ondes acoustiques incidentes étant obtenues en combinant linéairement n ondes incidentes élémentaires $E0_i(t)$ de façon correspondante à la formule :

$$\vec{E}(t) = H_c . \vec{E}0(t) \qquad (1)$$

où :

$H_c$ est une matrice carrée d'ordre n,

$\vec{E}(t) = [E_i(t)]$ est un vecteur ayant pour composantes les n ondes incidentes,

$\vec{E}0(t) = [E0_i(t)]$ est un vecteur ayant pour composantes les n ondes incidentes élémentaires, lesdites ondes incidentes élémentaires étant soit des ondes planes, soit des ondes divergentes, soit des ondes focalisées en différents points de l'espace présentant respectivement n fronts d'ondes différents,

chaque onde incidente élémentaire étant émise par une pluralité de transducteurs acoustiques du réseau (2)

de transducteurs acoustiques et balayant toute la zone d'observation,

(b) des moyens de réception (2, 13) adaptés pour capter par le réseau (2) de transducteurs acoustiques, successivement n ondes réverbérées R$_i$(t) qui sont réverbérées respectivement par le milieu à imager suite à l'émission des n ondes incidentes,

(c) des moyens de décodage (13) adaptés pour déterminer n ondes réverbérées élémentaires R0$_i$(t) en combinant linéairement les n ondes réverbérées R$_i$(t) captées, de façon correspondante à la formule :

$$\vec{R}0(t) = H_d . \vec{R}(t) \qquad (2)$$

où :

H$_d$ est une matrice carrée d'ordre n, les matrices H$_c$ et H$_d$ étant telles que Hc.Hd=D où D est une matrice diagonale d'ordre n dont tous les éléments diagonaux d$_{ii}$ sont non nuls,

$\vec{R}(t) = [R_i(t)]$ est un vecteur ayant pour composantes les n ondes réverbérées,

$\vec{R}0(t) = [R0_i(t)]$ est un vecteur ayant pour composantes les n ondes réverbérées élémentaires,

(d) des moyens de construction d'image (13), adaptés pour réaliser une image synthétique à partir des n ondes réverbérées élémentaires R0$_i$(t).

**Patentansprüche**

1. Verfahren zur akustischen Bildgebung, das die folgenden Schritte umfasst:

(a) einen Sende-/Empfangsschritt, bei dem eine Reihe von n einfallenden Schallwellen Ei(t) nacheinander in eine zu einem abzubildenden Medium (1) gehörende Beobachtungszone durch ein Array (2) von Schallwandlern gesendet werden und n zurückgeworfene Wellen R$_i$(t), die jeweils von dem abzubildenden Medium infolge der Aussendung der n einfallenden Wellen zurückgeworfen werden, von dem Array von Schallwandlern aufgenommen werden, wobei n eine natürliche Zahl von mindestens gleich 2 ist, wobei die einfallenden Schallwellen durch lineare Kombination von n einfallenden Elementarwellen EOi(t) entsprechend der folgenden Formel erhalten werden:

$$\vec{E}(t) = H_c . \vec{E}0(t) \qquad (1)$$

wobei:

H$_c$ eine quadratische Matrix der Ordnung n ist,

$\vec{E}(t) = [E_i(t)]$ ein Vektor ist, der als Komponenten die n einfallenden Wellen hat,

$\vec{E}0(t) = [E0_i(t)]$ ein Vektor ist, der als Komponenten die n einfallenden Elementarwellen hat, wobei die einfallenden Elementarwellen entweder ebene Wellen, divergente Wellen oder an verschiedenen Punkten im Raum fokussierte Wellen sind, die jeweils n verschiedene Wellenfronten haben, jede einfallende Elementarwelle von einer Vielzahl von Schallwandlern des Arrays (2) von Schallwandlern ausgesendet wird und den gesamten Beobachtungsbereich abtastet,

(b) einen Dekodierschritt, bei dem n zurückgeworfene Elementarwellen R0$_i$(t) durch lineare Kombination der n aufgenommenen zurückgeworfenen Wellen Ri(t) entsprechend der folgenden Formel bestimmt werden:

$$\vec{R}0(t) = H_d . \vec{R}(t) \qquad (2)$$

wobei:

H$_d$ eine quadratische Matrix der Ordnung n ist, wobei die Matrizen H$_c$ und H$_d$ so beschaffen sind, dass Hc.

Hd=D, wobei D eine Diagonalmatrix der Ordnung n ist, bei der alle Diagonalelemente $d_{ii}$ ungleich Null sind,
$\vec{R}(t) = [R_i(t)]$ ein Vektor ist, der als Komponenten die n zurückgeworfenen Wellen hat,
$\vec{R}0(t) = [R0_i(t)]$ ein Vektor ist, der als Komponenten die n zurückgeworfenen Elementarwellen hat.

(c) einen Bildkonstruktionsschritt, bei dem ein synthetisches Bild aus den n zurückgeworfenen Elementarwellen $R0_i(t)$ erstellt wird.

2. Verfahren nach Anspruch 1, wobei die Matrizen $H_c$ und $H_d$ so beschaffen sind, dass Hc.Hd=D, wobei D eine Diagonalmatrix der Ordnung n ist, bei der alle Diagonalelemente $d_{ii}$ größer als 1 sind.

3. Verfahren nach Anspruch 1 oder Anspruch 2, wobei n eine gerade natürliche Zahl ungleich Null ist.

4. Verfahren nach einem der vorstehenden Ansprüche, wobei die Matrix $H_c$ eine Hadamard-Matrix der Ordnung n ist und die Matrix $H_d$ die Transponierte der Matrix $H_c$ ist.

5. Verfahren nach einem der vorstehenden Ansprüche, wobei jede der Schallwellen durch eine Vielzahl von Zeitsignalen dargestellt wird, wobei jedes Zeitsignal die Schallwelle an einem zu dem Array von Wandlern gehörendem Wandler darstellt.

6. Verfahren nach einem der vorstehenden Ansprüche, wobei das abzubildende Medium (1) lebendes menschliches oder tierisches biologisches Gewebe umfasst.

7. Vorrichtung zur akustischen Bildgebung, die aufweist:

(a) Sendemittel (13, 2), die geeignet sind, eine Reihe von n einfallenden Schallwellen $E_i(t)$ nacheinander in eine zu einem abzubildenden Medium (1) gehörende Beobachtungszone durch ein Array (2) von Schallwandlern zu senden, wobei die einfallenden Schallwellen durch lineare Kombination von n einfallenden Elementarwellen $E0_i(t)$ entsprechend der folgenden Formel erhalten werden:

$$\vec{E}(t) = H_c . \vec{E}0(t) \qquad (1)$$

wobei:

$H_c$ eine quadratische Matrix der Ordnung n ist,
$\vec{E}(t) = [E_i(t)]$ ein Vektor ist, der als Komponenten die n einfallenden Wellen hat,
$\vec{E}0(t) = [E0_i(t)]$ ein Vektor ist, der als Komponenten die n einfallenden Elementarwellen hat, wobei die einfallenden Elementarwellen entweder ebene Wellen, divergente Wellen oder an verschiedenen Punkten im Raum fokussierte Wellen sind, die jeweils n verschiedene Wellenfronten haben, jede einfallende Elementarwelle von einer Vielzahl von Schallwandlern des Arrays (2) von Schallwandlern ausgesendet wird und den gesamten Beobachtungsbereich abtastet,

(b) Empfangsmittel (2, 13), die geeignet sind, n zurückgeworfene Wellen $R_i(t)$, die jeweils von dem abzubildenden Medium infolge der Aussendung der n einfallenden Wellen zurückgeworfen werden, durch das Array (2) von Schallwandlern aufzunehmen,
(c) Dekodiermittel (13), die geeignet sind, n zurückgeworfene Elementarwellen $R0_i(t)$ durch lineare Kombination der n aufgenommenen zurückgeworfenen Wellen $R_i(t)$ entsprechend der folgenden Formel zu bestimmen:

$$\vec{R}0(t) = H_d . \vec{R}(t) \qquad (2)$$

wobei:

$H_d$ eine quadratische Matrix der Ordnung n ist, wobei die Matrizen $H_c$ und $H_d$ so beschaffen sind, dass Hc.Hd=D, wobei D eine Diagonalmatrix der Ordnung n ist, bei der alle Diagonalelemente $d_{ii}$ ungleich Null sind,

$\vec{R}(t) = [R_i(t)]$ ein Vektor ist, der als Komponenten die n zurückgeworfenen Wellen hat,

$\vec{R}0(t) = [R0_i(t)]$ ein Vektor ist, der als Komponenten die n zurückgeworfenen Elementarwellen hat.

(d) Bildkonstruktionsmittel (13), die geeignet sind, ein synthetisches Bild aus den n zurückgeworfenen Elementarwellen ROi(t) zu erstellen.

**Claims**

1. An acoustic imaging method comprising the following steps:

(a) a transmission/reception step during which an array (2) of acoustic transducers successively transmits a series of n incident acoustic waves $E_i(t)$ in an area of observation that is part of a medium (1) to be imaged, and the array of acoustic transducers detects n reverberated waves $R_i(t)$ which are respectively reverberated by the medium to be imaged following the transmission of the n incident waves, n being a natural number at least equal to 2,
said incident acoustic waves being obtained by linearly combining n elemental incident waves $E0_i(t)$ in a manner corresponding to the formula:

$$\vec{E}(t) = H_c . \vec{E}0(t) \quad (1)$$

where:

$H_c$ is a square matrix of order n,
$\vec{E}(t) = [E_i(t)]$ is a vector having the n incident waves as components ,
$\vec{E}0(t) = [E0_i(t)]$ is a vector having the n elemental incident waves as components, said elemental incident waves being plane waves or diverging waves or waves focused at different points in space respectively having n different wave fronts,

each elemental incident wave being transmitted by a plurality of acoustic transducers of the array (2) of acoustic transducers and sweeping the entire area of observation,
(b) a decoding step during which n elemental reverberated waves $R0_i(t)$ are determined by linearly combining the n reverberated waves $R_i(t)$ detected, corresponding to the formula:

$$\vec{R}0(t) = H_d . \vec{R}(t) \quad (2)$$

where:

$H_d$ is a square matrix of order n, the matrices $H_c$ and $H_d$ being such that Hc.Hd = D where D is a diagonal matrix of order n in which all diagonal elements $d_{ii}$ are non-zero,
$\vec{R}(t) = [R,(t)]$ is a vector having the n reverberated waves as components ,
$\vec{R}0(t) = [R0_i(t)]$ is a vector having the n elemental reverberated waves as components,

(c) an image construction step during which a synthetic image is produced from the n elemental reverberated waves $R0_i(t)$ .

2. Method according to claim 1, wherein the matrices $H_c$ and $H_d$ are such that Hc.Hd = D, where D is a diagonal matrix of order n in which all diagonal elements $d_{ii}$ are greater than 1.

3. Method according to claim 1 or claim 2, wherein n is an even non-zero natural number.

4. Method according to any one of the preceding claims, wherein matrix Hc is a Hadamard matrix of order n and matrix $H_d$ is the transpose of matrix Hc.

5. Method according to any one of the preceding claims, wherein each of said acoustic waves is represented by a plurality of time signals, each time signal representing said acoustic wave at a transducer that is part of said transducer array.

6. Method according to any one of the preceding claims, wherein the medium (1) to be imaged comprises living human or animal biological tissue.

7. An acoustic imaging device, comprising:

(a)transmission means (13, 2) adapted for successively transmitting a series of n incident acoustic waves Ei(t) in an area of observation within a medium to be imaged, by means of an array (2) of acoustic transducers, said incident acoustic waves being obtained by linearly combining n elemental incident waves $E0_i(t)$ in a manner corresponding to the formula:

$$\vec{E}(t) = H_c.\vec{E}0(t) \quad (1)$$

where:

$H_c$ is a square matrix of order n,
$\vec{E}(t) = [E_i(t)]$ is a vector having the n incident waves as components ,
$\vec{E}0(t) = [E0_i(t)]$ is a vector having the n elemental incident waves as components, said elemental incident waves being plane waves or diverging waves or waves focused at different points in space respectively having n different wave fronts,

each elemental incident wave being transmitted by a plurality of acoustic transducers of the array (2) of acoustic transducers and sweeping the entire area of observation,
(b)reception means (2, 13) adapted for successive detection by the array (2) of acoustic transducers of n reverberated waves $R_i(t)$ which are respectively reverberated by the medium to be imaged following the transmission of the n incident waves,
(c)decoding means (13) adapted for determining n elemental reverberated waves $R0_i(t)$ by linearly combining the detected n reverberated waves $R_i(t)$, in a manner corresponding to the formula:

$$\vec{R}0(t) = H_d.\vec{R}(t) \quad (2)$$

where:

$H_d$ is a square matrix of order n, matrices $H_c$ and $H_d$ being such that Hc.Hd = D where D is a diagonal matrix of order n in which all diagonal elements $d_{ii}$ are non-zero,
$\vec{R}(t) = [R_i(t)]$ is a vector having the n reverberated waves as components ,
$\vec{R}0(t) = [R0_i(t)]$ is a vector having the n elemental reverberated waves as components,

(d) image construction means (13) adapted for creating a synthetic image from the n elemental reverberated waves $R0_i(t)$ .

# FIG. 1

# FIG. 2

FIG. 3

**FIG. 4a**

**FIG. 4b**

**FIG. 4c**

**FIG. 4d**

EP 3 198 301 B1

FIG. 5a

FIG. 5b

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2101191 A

**Littérature non-brevet citée dans la description**

- **MONTALDO et al.** Coherent plane-wave compounding for very high frame rate ultrasonography and transient elastography. *IEEE Trans Ultrason Ferroelectr Freq Control,* Mars 2009, vol. 56 (3), 489-506